# EUROPEAN PATENT APPLICATION

(11) **EP 3 000 315 A1**
(43) Date of publication of application: **30.03.2016**
(21) Application number: 14186569.1
(22) Date of filing: 26.09.2014
(51) Int. Cl.: A01H 3/04, A01H 5/10

(54) **Composition for inhibition of pod shatter**

(71) Applicant: Brandon Products Limited, Co. Kerry (IE)
(72) Inventor: The designation of the inventor has not yet been filed
(74) Representative: Purdy, Hugh Barry

(57) **Abstract**

The present invention relates to a composition for reducing pod shattering comprising a salt of phosphorous acid and to a method using said composition in the reduction of pod shatter.

## Description

The present invention relates to a composition for physiologically inhibiting pod shattering and to a method using the composition.

The crop plants oilseed rape and canola (*Brassica napus and Brassica rapa*) are important sources of seed-derived oils in Europe, North America, Australasia and elsewhere, the oils being used extensively in the food industry and for biodiesel production. Oilseed rape is a recently domesticated plant and retains some of the traits of

its wild ancestors which were useful as a wild plant but not as a commercial crop, including the splitting of the fruit (silique or "seed pod") when mature in order to effect seed dispersal.

In brassicas such as oilseed rape, two valves of a silique are fused to a central replum via a valve margin. This structure facilitates pod opening and the release of seeds. The splitting of the two valves of the silique results in shedding of the seeds and is called pod shattering. This process can occur before the crop is harvested and leads to average seed yield losses of 20-25 % and up to 70 %, as well as a concomitant fall in oil content of the harvested seeds.

Pod shattering is also exhibited by other brassica crops such as mustard, radish and turnip rape and by legumes such as pea, bean, clover, soybean as well as some other seed crops, for example, linseed.

Anything which delays harvesting increases risk of shattering losses, i.e. ripening variation within crop, lodging, poor weather at harvesting or drought during crop growth. The first-formed pods are most susceptible to shattering. These pods will be the most mature and will shatter first. Some of the negative consequences of pod shattering in, for example, oilseed rape are that more of the seeds harvested will be low in oil content which will result in a reduced price being paid to the farmer for the seed. Furthermore, steps are routinely taken to accelerate and synchronise pod maturity by killing the crop by windrowing or with a desiccant (usually a herbicide), but this has the added problem of generating a seed sample with a higher than acceptable percentage of green seeds which negatively affects oil quality and can lead to rejection of the seed sample. Additionally, late herbicide (i.e. desiccant) applications to crops increase the risk of detectable herbicide residues in harvested seed grain, potentially leading to breaches of maximum residue limits.

Flowering and hence pod formation in oilseed rape and other pod-forming crops is not synchronised. The lower pods on the main stem of an oilseed rape plant ripen first and have the highest oil content. Delaying harvesting (direct or after windrowing) until all the pods are mature runs the risk of the early-formed pods, i.e. those pods having the highest oil content, shattering before or during harvesting, with loss of seed, resulting in reduced seed yield and oil content.

Apart from direct losses of income from reduced seed yield and reduced price paid for low oil content seeds, pod shattering also results in additional indirect costs to the grower. The shed seed results in self-sown ("volunteer") oilseed rape plants growing in the next year's crop, which creates further expense due to the need for increased herbicide use. Such self-sown oilseed rape plants cause losses due to competition with subsequent crop and cause problems for farmers using reduced-tillage strategies such as no-till, zone-till, and strip tillage. Additionally, the self-sown plants provide food and shelter for slugs, which become a greater problem in the following crop, requiring increased molluscicide usage and resulting in reduced seed yields.

Commercial products for reducing pod shattering include pod sticker and pod sealant agents. These commercial products work by physically preventing the valves from separating, either by gluing the valves together or by forming a lattice around the pods. For example, US 4,447,984 discloses pod sticker agents based on pine resin, with the active ingredients being pinolene products based on di-1-*p*-menthene and US 2013/0061520 teaches low-viscosity carboxymethyl cellulose for sealing pods. Other more recent products of these types include "Iskay" (Interagro UK; carboxylated styrenebutadiene co-polymer preparation), "Pod-Stik" (DeSangosse; latex polymer) and "Pod- Ceal" (Miller Chemical and Fertiliser Corporation, USA; cyclohexane polymers).

However, pod sticker and pod sealant agents, which are contact in action, need to be applied when all the pods are near fully formed as their action is to coat the pods. This limits the application date of these products to within 6 weeks of harvest at the latest. It also means that high-volume spraying is necessary to reach all the pods, particularly in dense canopies. Side effects arise with commercial products incorporating pine resin, such as stickiness of the coating. The pod shattering reducing effect also wears off with time and can be dependent on weather conditions.

Other strategies used by growers to reduce pod shatter losses include physical and chemical desiccation. Physical desiccation entails windrowing or swathing, which involves cutting mature crop and allowing it to dry before harvesting the seed. Chemical desiccation involves spraying the crop just before maturity with contact herbicides such as diquat or the systemic herbicide glyphosate. Desiccation before maturity accelerates senescence but results in smaller seeds, lower oil content and possibly reduced oil quality as a result of excessive green seeds in the sample.

Pods need to be mature at harvest to achieve minimal green seed content. On the other hand, it is not appropriate to delay the harvest date because this could result in the crop being ready for harvest during poorer weather conditions which restricts the grower from harvesting the crop.

Considerable effort is being extended to develop shattering tolerant oilseed rape cultivars using conventional or genetic modification methodologies, but to date this has been without success.

It is an object of the present invention to provide a composition and method that seek to alleviate the aforementioned problems.

Thus according to a first aspect, the invention provides a composition for inhibiting pod shattering, the composition comprising a salt of phosphorous acid.

When applied to the growing plant, the composition according to the invention physiologically inhibits the splitting of the two valves of the pod. As a result, there is less premature pod shattering and more seed remains on the plant for harvest, resulting in increased seed yield. Treatment of plants with the composition maximises the recovery of the first-formed seeds and hence maximises the oil content of the seeds. Furthermore, there is a wider window of application, so that the composition according to the invention can be applied before pods are formed, unlike pod stickers or sealants.

The salt of phosphorous acid is preferably selected from among potassium, ammonium, sodium, aluminium, calcium, copper, zinc and magnesium salt and mixtures thereof. However, the salt of phosphorous acid is not limited to these particular salts. Any agriculturally acceptable salts of phosphorous acid are included within the scope of the invention.

The salts of phosphorous acid are commonly referred to as phosphites, also known as phosphonates, for example potassium hydrogen phosphonate (KH₂PO₃), dipotassium phosphonate (K₂HPO₃), ammonium phosphite ((NH₄)₂HPO₃), (NH₄)H₂PO₃)), sodium phosphite (Na₂HPO₃), aluminium phosphite (Al₂(HPO₃)₃), calcium phosphite (CaHPO₃), copper(I)phosphite (Cu^{I}H₂PO₃), copper(II)phosphite (Cu^{II}HPO₃)₂), zinc phosphite (ZnHPO₃), magnesium phosphite (MgHPO₃), or mixtures thereof.

Phosphite is known to induce resistance against crop pathogens which are caused by oomycetes. Oomycete diseases of oilseed rape include downy mildew, white rust and damping off. This effect is unrelated to the unexpected pod shatter reduction effect which is the subject of the present invention.

In a preferred embodiment, the composition comprises approximately 1 to 25 % w/v phosphonates, preferably 12 % w/v.

In a particularly preferred embodiment, the composition comprises 12 % w/v potassium phosphonates.

Preferably, the composition according to the invention is aqueous.

Unlike sticker and sealant agents, the effect of the composition according to the invention does not wear off with time as it is an internal, rather than external effect. As a result, the potential reduction in pod shattering is greater than with sticker and sealant agents.

Adjuvants which can be mixed with agrochemicals to increase their efficacy include spreaders (which reduce the surface tension to improve uptake of the agrochemical) such as organosilicones, fatty amine ethoxylates, alkylaryl ethoxylates and alcohol alkoxylates and stickers (which increase the period of rainfastness of the agrochemical) such as aapoe. Adjuvants work on the interaction between the leaf/pod surface (epidermis) and the agrochemical. The known pod sticker and sealant products also work on the outside of the pod, so adjuvants may interfere with the effectiveness of the sticker/sealant. Furthermore, the prescribed application date of pod stickers and sealants before desiccant application may result in the treatment preventing uptake of the desiccant into the plant. Unlike the known external products, the composition according to the invention can be mixed with adjuvants such as spreaders to increase efficacy.

The composition according to the invention reduces pod shattering in all crops which produce pods which dehisce. Dehiscence is the splitting at maturity along a natural line of weakness in a plant structure in order to release its contents. Plants which dehisce include oilseed rape (spring and winter varieties) and other brassica crops such as mustard, radish and turnip rape; linseed, and legumes such as field pea, lupin and field bean. Of these, oilseed rape is by far the most economically important crop.

Oilseed rape can be further segregated into double low varieties, high erucic acid rape (HEAR) varieties and high oleic, low linolenic fatty acid profile (HO, LL) varieties. Double low varieties are the most commonly grown in Ireland and typically have oil content of 43-44%. Double low varieties are grown for the food market and have low erucic acid content, less than 2% of measured fatty acid and low glucosinolates with less than 35 mmol/kg in the meal. HEAR varieties are more specialised and are generally grown for specialist markets including bio-fuels and are regarded as non-food crops. HO, LL varieties are healthier for human consumption and have high stable oil for the food processing industry.

Another major field crop which can benefit is soybean which suffers from pod shattering in tropical climates or where cycles of drying and wetting occur. This phenomenon is expected to become more important as climate change takes hold. One of the major pathogens of soybean is *Phytophthora sojae,* an oomycete, which causes root and stem rot of soybean and increased resistance to this disease is expected to occur as a side-effect of applying the composition of the present invention to soybean crops.

Without being bound by theory, the applicants believe that the composition according to the invention works by preventing dehiscence of the two valves of the silique. Plants sprayed as early as first flower, i.e. before the pods form, produce pods which are much less susceptible to pod shattering.

A broad range of plants may be treated with the compositions described herein. In particular, *Brassica, Glycine, Pisum, Raphanus, Sinapis, Gossypium* and *Vigna* plants may be treated.

Preferred plants to which the composition according to the invention may be applied are crop plants in which it is desired to inhibit dehiscence.

Particularly preferred plants to which the composition according to the invention may be applied are members of the Brassicaceae, such as rapeseed, or a member of the Fabaceae, such as a soybean, pea, lentil or bean plant.

The Fabaceae encompass both grain legumes such as soybean (*glycine*), pea, chickpea, moth bean, broad bean, kidney bean, lima bean, lentil, cowpea, dry bean and peanut and forage legumes such as alfalfa, lucerne, birdsfoot trefoil, clover, stylosanthes species and sainfoin. Preferred crop plants are those of the Brassicaceae family, in particular Brassica species selected from *Brassica napus, Brassica oleracea, Brassica campestris* (*Brassica rapa*)*, Brassica juncea, Brassica nigra* and *Brassica carinata.*

The composition according to the invention is most appropriate for treatment of brassica crops grown for their seed, such as *napus, nigra, juncea, carinata,* as well as for production of brassica seed for propagation.

According to a further aspect, the invention provides a method for inhibiting pod shattering or increasing grain yield in a plant, the method comprising the application of a composition as described herein to a growing plant. Increasing grain yield should be understood to mean an increase in the yield of grain obtained from the treated plant compared with an untreated control plant.

By growing plant is meant a plant which is in a growth stage selected from among the vegetative stages of rosette stage, bud stage and the reproductive stages of flower, ripening and maturation, preferably from rosette stage to green pod stage.

The composition according to the invention is systemic in action thus a low-volume application is possible and can be applied from first open flower onward, before pods are produced, giving a much wider window of application. Many growers do not like to spray oilseed rape crops close to maturity because of the risk of physical disturbance of the crop such as damage to leaves and stems by machinery causing increased pod shattering. Also the large leaf canopy of crops close to maturity requires higher volume sprays.

The composition is typically applied to growing plants. Preferably, the composition is applied at one or more of the following three stages of growth according to the BBCH-scale (Biologische Bundesanstalt, Bundessortenamt and Chemical Industry; Lancashire et al., 1991): Four leaf stage (BBCH-14), second internode stage (BBCH-32), and first flower opened stage (BBCH-60). However, application of the composition may occur at any other principal growth stage according to the BBCH scale, e.g. any of stages 10-19, 21-29, 31-39, 40-49, 51-59 and 60-69 Particularly preferably, the composition is applied at each of the four leaf stage (BBCH-14), second internode stage (BBCH-32) and first flower opened stage (BBCH-60). For soybean plants, the composition is preferably applied at each of the four leaf stage (BBCH-14), 3rd side shoot of first order visible stage (BBCH-24) and first flower opened stage (BBCH-60). For gossypium plants the composition is preferably applied at each of the four leaf stage (BBCH-14), 50% of plants meet between rows stage (BBCH-35) and first flower opened stage (BBCH-60) and particularly preferably when the pods have attained their final size stage, preferably when 50 % of pods have attained their final size (BBCH-75).

Alternatively, or additionally, the composition is applied at a growth stage of green pod, when 50% of pods are ripe (BBCH-85). This can be done when farmers do not only want to use the composition prophylactically, but when conditions look to be conducive to pod shattering.

Preferred application techniques include foliar spray, soil drench and pre or in furrow seed treatment.

The compositions and methods of the present invention can be used to reduce pod shatter and seed loss, while maintaining at the same time an agronomically relevant threshability of the pods, enabling the pods to be opened along the dehiscence zone by applying physical force via the thresher.

### Examples

### Example 1

### Preparation of preferred composition

Phosphorous (phosphonic) acid is neutralised in aqueous solution with potassium hydroxide to produce potassium hydrogen phosphonate (KH₂PO₃) and dipotassium phosphonate (K₂HPO₃). This composition is commonly referred to as potassium phosphite. Both salts are produced when phosphorous acid is neutralised in aqueous solution with potassium hydroxide according to the reactions:

H₃PO₃ + KOH = KH₂PO₃ + H₂O and

KH₂PO₃ + KOH = K₂HPO₃ + H₂O

An aqueous solution containing 7.4 kg of potassium hydroxide or potassium carbonate is added slowly with stirring to an aqueous solution containing 8.2 kg of phosphonic acid in a jacketed reaction vessel with constant cooling to produce a solution (30 litres) containing a mixture of approximately 8 kg of potassium hydrogen phosphonate and 7 kg of dipotassium phosphonate, i.e. containing approximately 50 % w/v dipotassium phosphonate.

### Example 2

### Treatment of spring oil seed rape

The solution according to Example 1 is used in a field trial is located near Grenfell, NSW, Australia at a site characterised by Brown Clay Loamy soil with pH 5.1.

The field trial is constructed using a completely randomised design, 6 replicates per treatment with each plot measuring 100 - 150 m² size, with 12.5 cm row spacing, with seeds (Goldcrop Delight seed variety) sown to a depth of 2.5 cm and a seeding rate of 4.0 kg/ha.

The solution according to Example 1 is applied by spraying onto foliage at the following growth stages: Four leaf stage (BBCH-14), second internode (BBCH-32) and when the first flower opens (BBCH-60), at a rate of 1.5 litres per hectare in 600 litres of water.

Control plots are sprayed with water.

The crops also receive standard fertiliser applications, pesticidal and fungicidal treatments. The crop is chemically desiccated before maturation (approximately 14 days before harvest) by applying a foliar spray of systemic herbicide glyphosate. At maturation, the plots are harvested directly with a combine harvester.

Foliar application with potassium phosphonate significantly increases grain yield when compared to the control.

It will of course be understood that the invention is not limited to the specific details as herein described, which are given by way of example only, and that various alterations and modifications are possible without departing from the scope of the invention as defined in the appended claims.

## Claims

1. A composition for use in reducing pod shattering, the composition comprising a salt of phosphorous acid.

2. The composition for use in reducing pod shattering as claimed in claim 1, wherein the salt is selected from among potassium, ammonium, sodium, aluminium, calcium, copper, zinc and magnesium salt and mixtures thereof.

3. A method for reducing pod shattering comprising the application of the composition as defined in claim 1 or claim 2 to a plant, wherein the composition is applied at one or more stages of growth according to the BBCH-scale.

4. The method for reducing pod shattering as claimed in claim 3, wherein the composition is applied to the plant at one or more of the four leaf stage (BBCH-14), second internode stage (BBCH-32) and first flower opened stage (BBCH-60).

5. The method for reducing pod shattering as claimed in claim 3 or claim 4, wherein the composition is applied to the plant at each of the four leaf stage (BBCH-14), second internode stage (BBCH-32) and first flower opened stage (BBCH-60).

6. The method for reducing pod shattering as claimed in any one of claims 3 to 5, wherein the composition is applied to the plant at a green pod growth stage, preferably when 50% of pods are ripe (BBCH-85).

7. The method for reducing pod shattering as claimed in any one of claims 3 to 6, wherein the composition is applied to a brassica plant.

8. The method for reducing pod shattering as claimed in claim 7, wherein the composition is applied to oilseed rape.

9. The method for reducing pod shattering as claimed in claim 7, wherein the composition is applied to a gossypium plant.

10. The method for reducing pod shattering as claimed in claim 9, wherein the composition is applied to the gossypium plant at one or more of the four leaf stage (BBCH-14), 50% of plants meet between rows stage (BBCH-35) and first flower opened stage (BBCH-60).

11. The method for reducing pod shattering as claimed in claim 9 or claim 10, wherein the composition is applied to the gossypium plant when the pods have attained their final size stage, preferably when 50 % of pods have attained their final size (BBCH-75).

12. The method for reducing pod shattering as claimed in any one of claims 3 to 6, wherein the composition is applied to a soybean plant.

13. The method for reducing pod shattering as claimed in any one of claims 3 to 12, wherein the composition is applied by spraying onto foliage.

14. The method for reducing pod shattering as claimed in any one of claims 3 to 13, wherein the plant is also treated with one or more applications selected from among fertiliser applications, pesticidal treatments and fungicidal treatments.

15. The method for reducing pod shattering as claimed in any one of claims 3 to 14, wherein the plant is chemically desiccated before maturation, preferably by applying a foliar spray of systemic herbicide, e.g. glyphosate.

16. Use of a composition comprising a salt of phosphorous acid for reducing pod shattering.

17. A method for reducing pod shattering substantially as herein described.

18. A composition for use in reducing pod shattering substantially as herein described.
